# EUROPEAN PATENT SPECIFICATION

(11) **EP 2 751 729 B1**
(45) Date of publication and mention of the grant of the patent: **21.10.2020**
(21) Application number: 12827479.2
(22) Date of filing: 30.08.2012
(51) Int. Cl.: G16B 50/00, G16B 45/00, G16B 5/00, G16B 25/00, G16B 30/00, G06Q 30/06

(54) **SYSTEM FOR POLYNUCLEOTIDE CONSTRUCT DESIGN, VISUALIZATION AND TRANSACTIONS TO MANUFACTURE THE SAME**
SYSTEM ZUM ENTWURF UND ZUR VISUALISIERUNG VON POLYNUKLEOTIDKONSTRUKTEN UND TRANSAKTIONEN ZU IHRER HERSTELLUNG
SYSTÈME DE CONCEPTION, DE VISUALISATION ET DE TRANSACTION DE CONSTRUCTIONS POLYNUCLÉOTIDIQUES EN VUE DE LEUR PRODUCTION

(30) Priority: 01.09.2011 US 201161530384 P; 30.11.2011 US 201161565435 P; 02.02.2012 US 201261594323 P; 02.02.2012 US 201261594320 P
(43) Date of publication of application: 09.07.2014
(73) Proprietor: Genome Compiler Corporation, San Francisco, CA 94158 (US)
(72) Inventor: AMIRAV-DRORI, Omri, 45269 Hod-Hasharon (IL); BEN MOSHE, Nir, 62744 Tel Aviv (IL)
(74) Representative: Puschmann Borchert Kaiser Klettner Patentanwälte Partnerschaft mbB
(86) International application number: PCT/IL2012/000326
(87) International publication number: WO 2013/030827

(56) References cited:
- WO-A1-02/27638
- WO-A2-01/56216
- WO-A2-2010/141433
- US-A1- 2007 043 516
- US-A1- 2007 043 516
- NOURI A, CHYBA C F: "Proliferation-resistant biotechnology: an approach to improve biological security", NATURE BIOTECHNOLOGY, vol. 27, no. 3, 1 March 2009 (2009-03-01), pages 234-236, XP055577207, United States

## Description

The present invention relates in generally to a computerized system for designing and synthesizing nucleic acid sequences for gene expression according to claim 1.

The use of synthetic nucleic acid constructs for critical applications has never been greater. Synthetic nucleic acids are used in a variety of fields from life sciences research to applications involving alternative sources of energy and beyond.

For example, almost everything that our civilization needs or creates starts with finite resources of energy, such as oil, coal and natural gas with growing demand and dwindling supply. The prices of a great majority of commodities are going up. The need for renewable sources of biomass as an energy source is expanding. The use of synthetic nucleic acids to design living organisms for resources such as energy and food will be widespread into the future.

To emphasize the magnitude of the problem and the opportunity, the department of energy released a 2004 report showing 120 high- value chemicals that can be bio-manufactured, each with a more than one billion dollar market size. The synthetic nucleic acid market for the source code of life is more than five hundred million dollars today.

With the National Institutes of Health spending more than 1.5 billion dollars on manipulating DNA in 2008, this market is projected to grow to 3 billion dollars in 2015, with the market for synthetic biological products estimated at 4.5 billion dollars that same year.

The creation of useful living organisms strongly relies on synthetic nucleic acids. The design and manipulation of nucleic acids traditionally has comprised the use of molecular biology, which is known to be slow and error prone. The design of nucleic acids in a way that resembles typing in a word processor would increase the ease and speed of editing, generating and synthesizing nucleic acid sequences as large as a genome.

US 2007/0435516 A1 discloses computer systems, computer program products and methods for designing oligonucleotides are provided. A set of sequence elements is defined. Each sequence element represents an amino acid sequence segment or a nucleic acid sequence segment. The set of sequence elements collectively represent a design nucleic acid sequence. The set of sequence elements are displayed as a plurality icons in a linear or a near linear arrangement such that each respective icon in the plurality of icons uniquely represents a corresponding sequence element in the set of sequence elements. In this representation, neighboring icons in the plurality of icons represent neighboring sequence elements in the set of sequence elements. Each respective icon in the plurality of icons depicts a directional property for the corresponding sequence element in the set of sequence elements.; An oligonucleotide selection module is used to identify oligonucleotides in the design nucleic acid sequence. In particular, US 2007/0435516 A1 discloses computer program product for use in conjunction with a computer system, the computer program product comprising a computer readable storage medium and a computer program mechanism embedded therein, the computer program mechanism comprising instructions for representing a set of sequence elements, each sequence element representing an amino acid sequence segment or a nucleic acid sequence segment, wherein the set of sequence elements are collectively represented by a design nucleic acid sequence, wherein said instructions for representing said set of sequence elements comprise instructions for displaying a plurality of icons in a linear or a near linear arrangement, each respective icon in said plurality of icons uniquely representing a corresponding sequence element in said set of sequence elements such that neighboring icons in said plurality of icons represent neighboring sequence elements in said plurality of sequence elements, and each said respective icon in said plurality of icons depicts a directional property for the corresponding sequence element in said set of sequence elements.

WO01/56216 A2 discloses systems, methods, and computer program products are described that process inquiries or orders regarding purchase of biological devices, substances, or related reagents. In some implementations, a user selects probe-set identifiers that identify microarray probe sets capable of enabling detection of biological molecules. Corresponding genes or EST's are identified and are correlated with related product data, which is provided to the user. Further, the user may select products for purchase based on the product data. If so, the user's account may be adjusted based on the purchase order. In the same or other implementations, a local genomic database is periodically updated. In response to a user selection of probe-set identifiers, data related to corresponding genes or EST's is provided to the user from the local genomic database. In particular, WO01/56216 A2 discloses a system for providing data related to one or more genes or EST's, wherein each gene or EST has at least one corresponding probe set identified by a probe-set identifier and capable of enabling detection of a biological molecule, comprising an input manager constructed and arranged to receive from a user a selection of a first set of one or more of the probe-set identifiers, a gene determiner constructed and arranged to identify a first set of one or more genes or EST's corresponding to the probe sets identified by the first set of probe-set identifiers, a correlator constructed and arranged to correlate the first set of genes or EST's with a first set of one or more data, and an output manager constructed and arranged to provide the first set of data to the user.

A computerized system for designing and synthesizing nucleic acid sequences for gene expression is known from the document "Proliferation-resistant biotechnology: approach to improve biological security", by Nouri A, Chyby C F, Nature Biotechnology, vol. 27, no. 3, 1 March 2009 (2009-03-01), pages 234-236, XP055577207, which is considered as the closest prior art.

There is a need for treating the design and synthesis of nucleic acid sequences, leveraging the tools of information technology, for efficient design and manufacturing of synthetic nucleic acids at a lower cost, with greater ease, reliability, and speed, while detecting when harmful sequences are designed and notifying the user of such detection.

Therefore a computerized system for designing and synthesizing nucleic acid sequences for gene expression according the present invention is defined in claim 1.

Further embodiments of the invention are defined in dependent claims 2 to 8.

According to the invention a computerized system for designing and synthesizing nucleic acid sequences for gene expression comprises; (A) a server [103] for hosting a database [106], wherein the database comprises a plurality of entries, each entry comprising a nucleic acid sequence encoding for a harmful gene expression product, wherein the nucleic acid sequence encoding for the harmful gene expression product is from a bacterial genome or a viral genome, (B) a network connection [102] and (C) a computer readable medium [101]; wherein said system is operating in a method of: (i) visualizing biological information in an interactive user interface relating to design instructions; (ii) designing nucleic acid sequences; (iii) automatically flagging when the nucleic acid sequence encoding for the harmful gene expression product is included in the nucleic acid sequence for at least one gene; (iv) automatically modifying the interactive user interface to notify when the nucleic acid sequence encoding for the harmful gene expression product is detected in the nucleic acid sequence for at least one gene; (v) providing an alternative alteration of the designed nucleic acid sequences, wherein the alternative alteration does not encode for the nucleic acid sequence encoding for the harmful gene expression product, and (vi) synthesizing a nucleic acid encoding for the alternative alteration of the designed nucleic acid sequences.

It is a further object of the present invention to disclose the system as defined above, wherein said design module is further adapted for optimizing said designed nucleic acid construct according to various requirements, wherein said various requirements are selected from a group consisting of: optimizing codon usage, optimizing GC content, optimizing CpG content, avoiding problems associated with DNA motifs, avoiding problems associated with sequence repeats, avoiding problems associated with inverse complementary sequence repeats and any combination thereof.

It is a further object of the present invention to disclose the system as defined above, wherein said system is further configured to compare designed nucleic acid constructs to said database [106] entries through sequence alignment.

It is a further object of the present invention to disclose the system as defined above, wherein said system is further configured to provide output with alternative alteration of said designed nucleic acid construct.

It is a further object of the present invention to disclose the system as defined above, wherein said system is further configured for optimizing said synthesis of the nucleic acid constructs.

It is a further object of the present invention to disclose the system as defined above, wherein said optimizing is based on cost, ease, and/or speed.

It is a further object of the present invention to disclose the system as defined above, wherein said interactive user interface is further configured to display various modules , wherein said various modules are selected from a group consisting of nucleic acid design, error checking, codon optimization, metabolic product design, visual representations of biological information with dynamic zoom, nucleic acid synthesis, transactions regarding ordering and/or purchasing synthesized nucleic acids, user review and ranking of nucleic acid.

It is a further object of the present invention to disclose the system as defined above, wherein said system is further configured for design of expressions constructs based on a list of design rules.

The following description is provided in order to enable any person skilled in the art to make use of the invention and sets forth the best modes contemplated by the inventor of carrying out this invention. Various modifications, however, are adapted to remain apparent to those skilled in the art, since the generic principles of the present invention have been defined specifically to provide a computerized system for designing nucleic acid sequences for gene expression and methods thereof.

A few preferred embodiments will now be described, by way of non-limiting example only, with reference to be accompanying drawings, in which:
Fig.1 describes a detecting module for detecting designated sequences including harmful sequences;
Fig.2 shows a flowchart of a detecting module for detecting designated sequences including harmful sequences; and
Fig.3 describes a compilation module adapted for functionally checking a designed nucleic acid sequences against a list of predetermined rules.

The following description relates to a systems and methods for the design, synthesis, purchase, sale and/or delivery of nucleic acid constructs and related materials. In one aspect, the description relates to a software application that can be installed in a computerized system, a phone, a handheld device or may be accessed by conventional similar means at a remote location.

The software application allows users to generate nucleic acid constructs according to user criteria / rules, such as an expression system, a metabolic pathway, mutation information and the like. The software application can have a variety of modules.

The modules can be made available to a user according to software package criteria, such as payment level, user proficiency, user access level or any other suitable package criteria.

The systems and methods described herein further relate to error recognition and error correction in nucleic acid constructs. Various embodiments of the invention further relate to centralized or distributed ranking, rating and/or review of nucleic acid constructs.

Transaction and pricing methods for the purchase of user-designed or pre-stocked nucleic acid constructs are described and can be based on various criteria, such as the availability of the sequence, the synthesis method required to manufacture the nucleic acid construct, the uses of the nucleic acid construct (e.g. generation of any metabolic products) or any other suitable criteria.

The methods described herein relate to the synthesis methods of the nucleic acid constructs, such as de novo synthesis, synthesis from pre-existing parts of a construct or any other suitable synthesis method. Preexisting parts can be located at one or more facilities, based on the nucleic acid construct to be synthesized. The synthesis of the nucleic acid construct can be optimized by picking a strategy of combined synthesis.

Preferably, the methods and systems allow for reading, writing and designing nucleic acids, for example, in the context of individual open reading frames (ORF), mobile genetic elements (such as plasmids), complete genomes and/or any suitable context known in the art.

Preferably, a software tool allows for the creation of genetic element components from databases comprising known sequenced genes from a variety of species. Related databases storing and related functions of genetic elements can also be accessed to pair genetic elements with one or more function. Other relevant information, such as organism information, homologs, orthologs or any other suitable information can be further accessed using available suitable databases known in the art.

Preferably, a visual user interface operably linked to the systems described herein represents relevant information for nucleic acid design.

Preferably, visual icons representing relevant information and functionalities can be selected, dragged and/or dropped using the user interface to actuate effects during nucleic acid editing and/or design. For example, an icon representing a selected metabolic product can be selected and dragged into an icon representing a desired organism to initiate an algorithm to design a nucleic acid construct, for example a genome and/or plasmid, that would most closely resemble the desired metabolic effect.

Preferably, the software can further output protocols to insert designed nucleic acids into the organism and/or to functionally express the designed constructs in selected organisms, such as induction protocols.

Preferably, the generated nucleic acid constructs comprise sequences for affecting the expression of functional genes encoding desired gene products.

Preferably, the methods and systems relate to software and/or services enabling users to customize genome features.

Preferably, the software and/or services are accessed over the internet.

Preferably, upon designing a nucleic acid constructs, users are allowed to order and/or purchase products that are related to the designed nucleic acid.

Preferably, the ordered and/or purchased products comprise a kit comprising an isolated synthetic preparation of a designed nucleic acid construct, e.g. a plasmid, and any other reagents and protocols for the purpose of inserting and/or expressing the designed construct in a desired organism.

### Exemplary Module 1 USER INTERFACE AND VARIOUS MODULES OF THE APPLICATION

The methods and systems as described herein are carried out using a user interface. The user interface can display various modules with associated functionalities, such as nucleic acid design, error checking, codon optimization, metabolic product design, visual representations of biological information with dynamic zoom, nucleic acid synthesis, transactions regarding ordering and/or purchasing synthesized nucleic acids, user review and ranking of nucleic acid constructs or any other suitable module with a functionality described herein or otherwise known in the art.

Preferably, the systems and methods can be made available as different versions. For example, different pay grades can give access to different modules and/or level of functionality within a module. Preferably the access level may depend on a user type.

Preferably, user can be matched with a skill-level. The skill-level of the user may lead to access to different versions of the systems and methods.

### Exemplary Module 2: DETECTING DESIGNED SEQUENCES COMPRISING HARMFUL OR TOXIC SEQUENCES

The system and method as described herein relates to algorithms for detection of harmful sequences, such as nucleic acid constructs capable of encoding portions of viruses or toxins.

An alert mechanism can be deployed to alert one or more authorized users of the system, when such sequences are designed, ordered and/or purchased.

Preferably, a database is compiled representing a list of harmful products, such as toxins, viruses, bacteria, pathogens, and more. The database can contain nucleic acid sequences strongly associated with harmful products.

Preferably, a risk level is stored in the database for a portion or all of the entries.

Preferably, a nucleic acid construct can be flagged for alert if a portion of the nucleic acid is above a threshold level of alignment with one or more entries in the database.

Preferably, the threshold level is set to be the same for all members of the database.

Preferably, the threshold level can be adjusted, for example according to the risk level associated with a particular database entry.

Preferably, the price of the nucleic acid construct can be adjusted, for example, a higher price can be assigned to a nucleic acid construct associated with a more harmful entry.

Preferably, various alert levels trigger price increases for the nucleic acid construct. For example, a linear, exponential or any other suitable functional relationship can be used between a price increase and the % similarity and or identity between the nucleic acid construct and one or more entries in the harmful sequence database.

Preferably, a price increase is triggered only after a base level of % similarity and/or identity. The database can be constructed de novo or by compiling public or private information. For example, content from National Select Agent Registry (CDC; http://www.selectagents.gov/Select%20Agents%20and%20Toxins%20List.html), The Australia Group (http://www.australiagroup.net/enlbiological_agents.html), World Health Organization (http://www.who.ini csr/disease/smallpox/Summaryrecommendati onsMay08.pdf), the summary webpage by the International Gene Synthesis Consortium (IGSC; http://www.genesynthesisconsortium.org/Harmonized_Screening_Protocol.html) or any other suitable depository can be compiled to construct a database.

Preferably, a portion or the entirety of a designed nucleic acid construct can be aligned against entries in a harmful sequences database.

Preferably, one or more annotated sequence elements in a designed nucleic acid construct is aligned against the entries in a database of harmful sequences.

The entirety of the nucleic acid constructs can be additionally aligned against the database entries, when triggered by a certain event such as a purchase order.

Preferably, the alignment comprises % similarity, identity or both.

Preferably, a similarity or identity of greater than 50%, 60%, 70%, 80%, 90%, 95% or more to one or more entries in the harmful sequence database results in the flagging of the nucleic acid construct.

Preferably, several thresholds can be deployed for higher levels of alert, for example: low, medium or high alerts.

Certain alert levels can result in the deployment of an automated alert, while others may require the attention of a human first. For example, a high alert level may generate an automated alert, and a medium alert level may be further processed upon initial review by a technician.

Alerts can be designed in a variety of ways.

Preferably, best practice methods can be followed from a specialized institution, for example best practice methods from IGSC can be implemented.

Preferably, the methods and systems of the invention comprises an algorithm for detection of harmful sequences (virus toxins) comprising: (a) a database of harmful sequences, (b). sequence alignment with the user "ordered" sequence and (c) a smart threshold for flagging harmful sequences with the corresponding price increase for the part and notification of authorities.

### Exemplary Module 3: OPTIMIZING NUCLEIC ACID CONSTRUCTS FOR VARIOUS USES

The methods and systems as described herein related to the design of nucleic acid constructs by allowing the editing of a nucleic acid sequence and the use of controls such as alteration of methylation sites, protein level changes or metabolic level changes.

Preferably, editing of nucleic acids comprises editing for the incorporation of non- natural amino acid or other substitute molecule coding sequences in relevant translation systems known in the art.

Preferably, the substitute molecules comprise heavy metals, markers, encoded data or any other suitable molecule known in the art.

According to the systems and methods as described herein, a user can create a nucleic acid design from genetic elements that are well known in the art, such as transcription promoters, ribosome binding sites, genes, and terminators. The designed nucleic acid constructs can be saved and ordered using the tools and systems described herein.

One technique for the preparation and synthesis of proteins is the cloning and expression of the gene sequence corresponding to the protein in heterologous systems, e^g. Escherichia coli or yeast. Naturally occurring genes are, however, frequently suboptimal for this purpose. Since in a DNA sequence expressing a protein in each case one triplet of bases (codon) expresses one amino acid, it is possible for an artificial DNA sequence for expression of the desired protein to be synthesized and to be used for cloning and expression of the protein. One problem with this procedure is that a predefined amino acid sequence does not correspond to a unique nucleotide sequence. This is referred to as the degeneracy of the genetic code. The frequency with which different organisms use codons for expressing an amino acid differs (called the codon usage). There is ordinarily in a given organism one codon which is predominantly used and one or more codons which are used with comparatively low frequency by the organism for expressing the corresponding amino acid. Since the synthesized nucleotide sequence is to be used in a particular organism, the choice of the codons ought to be adapted to the codon usage of the appropriate organism.

A further important variable is the GC content (content of the bases guanine and cytosine in a sequence).

Further factors which may influence the result of expression are DNA motifs and repeats or inverse complementary repeats in the base sequence.

Certain base sequences produce in a given organism certain functions which may not be desired within a coding sequence. Examples are cis-active sequence motifs such as splice sites or transcription terminators. The unintentional presence of a particular motif may reduce or entirely suppress expression or even have a toxic effect on the host organism.

Sequence repeats may lead to lower genetic stability and impede the synthesis of repetitive segments owing to the risk of incorrect hybridizations.

Inverse complementary repeats may lead to the formation of unwanted secondary structures at the RNA level or cruciform structures at the DNA level, which impede transcription and lead to genetic instability, or may have an adverse effect on translation efficiency.

A synthetic gene can be optimized in relation to the codon usage and the GC content and, on the other hand, substantially avoid the problems associated with DNA motifs and sequence repeats and inverse complementary sequence repeats.

These requirements cannot, however, ordinarily be satisfied simultaneously and in an optimal manner. For example, optimization to optimal codon usage may lead to a highly repetitive sequence and a considerable difference from the desired GC content.

The aim therefore is to reach a compromise which is as optimal as possible between satisfying the various requirements. However, the large number of amino acids in a protein leads to a combinatorial explosion of the number of possible DNA sequences which, in principle, are able to express the desired protein.

Various computer-assisted methods can be utilized for ascertaining an optimal codon sequence.

For example, a nucleotide sequence can be optimized for the expression of a protein using the nucleic acid optimization methods in organisms such as, viruses, especially vaccinia viruses; prokaryotes, especially Escherichia coli, Caulobacter cresentus, Bacillus subtilis, Mycobacterium spec; yeasts, especially Saccharomyces cerevisiae, Schizosaccharomyces pombe, Pichia pastoris, Pichia angusta; insects, especially Sprodoptera frugiperda, Drosophila spec; mammals, especially Homo sapiens, Macaca mulata, Mus musculus, Bos taurus, Capra hircus, Ovis aries, Oryctolagus cuniculus, Rattus norvegicus , Chinese hamster ovary; monocotyledonous plants, especially Oryza sativa, Zea mays, Triticum aestivum; dicotyledonous plants, especially Glycin max, Gossypium hirsutum, Nicotiana tabacum, Arabidopsis thaliana, Solanum tuberosum.

The expression of certain proteins may benefit particularly from nucleic acid optimization of their coding sequences.

Generally, a wide range of proteins are targets for nucleic acid optimization of their coding sequences, for example, enzymes, especially polymerases, endonucleases, ligases, lipases, proteases, kinases, phosphatases, topoisomerases; cytokines, chemokines, transcription factors, oncogenes; proteins from thermophilic organisms, from cryophilic organisms, from halophilic organisms, from acidophilic organisms, from basophilic organisms; proteins with repetitive sequence elements, especially structural proteins; human antigens, especially tumor antigens, tumor markers, autoimmune antigens, diagnostic markers; viral antigens, especially from HAV, HBV, HCV, HIV, SIV, FIV, HPV, rinoviruses, influenza viruses, herpesviruses, poliomaviruses, hendra virus, dengue virus, AAV, adenoviruses, HTLV, RSV; antigens of protozoa and/or disease- causing parasites, especially those causing malaria, leishmania; trypanosoma, toxoplasmas, amoeba; antigens of disease-causing bacteria or bacterial pathogens, especially of the genera Chlamydia, staphylococci, Klebsiella, Streptococcus, Salmonella, Listeria, Borrelia, Escherichia coli; antigens of organisms of safety level L4, especially Bacillus anthracis , Ebola virus, Marburg virus, poxviruses.

Given these criteria, various algorithms for optimizing nucleic acid constructs are well known in the art and are further described in US Pat. Pub. 2007/0141557.

Nucleic acid constructs can further be optimized to modulate gene expression by controlling the CpG content. The optimization in various embodiments relates to varying the proportion of CpG dinucleo tides, which correlate with a change in the level of expression of an encoded gene product.

The expression system may be a cell, or a cell-free system or in vitro system. A prokaryotic or eukaryotic expression system may be used, though a eukaryotic expression system is preferably employed. Suitable expression systems include e.g. bacterial cells, insect cells, e.g. Baculovirus expression systems, SF9 cells, Drosophila-Schneider cells, plant cells, yeasts, e.g. Saccharomyces Cerevisiae, Pichia angusta, Pichia pastoris and the like; as well as also algae, e.g. Chlamydomonas. Examples of possible plant expression systems include Arabidopsis thaliana, Zea mays (com), Nicotiana tobacco (tobacco), Oryza sativa (rice), Hordeum vulgare (barley), Glicine max (soya), Brassica sp. (cabbage) and the like. Preferably vertebrate cells are used, in particular mammalian cells, especially human cells, in particular somatic cells and no germ line cells.

The expression system can be a system or a cell with a low level of methylation, i.e. substantially no de novo methylation takes place. On the other hand it is also possible to use the nucleic acid optimization methods, for example ones that relate to varying the proportion of CpG dinucleotides, for the production of transgenic non-human organisms, in particular plants and animals.

In addition, the expression of the corresponding gene can be negatively influenced (smaller number of CpG) or positively influenced (increased number of CpG) by suitably choosing the number of CpG dinucleotides, and may even exceed the expression rates that can be achieved with a codon-optimised gene. The expression may even be raised under certain circumstances, for example, if the increase in the number of CpG dinucleotides takes place at the expense of the RNA and codon optimisation.

Preferably, no CpG islands are introduced in the modification of the target nucleic acid sequence.

Preferably, the modified target nucleic acid sequence is not associated with CpG islands. A correlation between the level of expression and the number of CpG dinucleotides can generally dictate nucleic acid optimization.

For the expression of genes these modifications are preferably introduced so that the coded amino acid sequence is not altered.

In the ideal case only the nucleic acid sequence of a corresponding gene should influence its level of expression. Since the genetic code is degenerate, there is the possibility, for a specific amino acid sequence, of choosing a plurality of corresponding nucleic acid sequences.

The number of CpG dinucleotides compared to the sequence of the target nucleic acid sequence to be expressed can be increased or reduced, depending on the desired level of expression, by at least 2, preferably at least 3, more preferably at least 5, still more preferably at least 8, yet more preferably at least 10, even more preferably by at least 15, and up to 20 or more, especially by 30- 50 or even up to 100 or more, depending on the length of the target nucleic acid sequence to be expressed.

Preferably, the number of CpG dinucleotides compared to the sequence of the target nucleic acid to be expressed is raised or lowered by at least 10%, 20%, 50%, 100%, or 200%, or by a factor of at least 3, 5, 5, 10, 20 or more.

It is possible to make use of the degeneracy of the genetic code so that preferably the maximum number of CpG dinucleotides is introduced or eliminated without having to alter the amino acid sequence of the target nucleic acid sequence to be expressed. The maximum number of CpG dinucleotides to be introduced is limited by the variation possibilities of the degenerated codon of a predetermined amino acid sequence. On the other hand, if desired the number of CpG dinucleotides may be increased still further, even if the corresponding amino acid sequence is thereby altered. In these cases, additional suitable protocols can make sure that care is taken to ensure that the function of the gene product, such as a peptide or protein is not interfered with.

In addition to increasing or reducing the number of CpG dinucleotides, additional types of nucleic acid optimization can be carried out, so that either the gene expression is promoted or inhibited, or is reduced. For example, insertion or removal of motifs can influence the gene expression, such as secondary structure- stabilising sequences, regions with raised self-homology, regions with raised homology with respect to the natural gene, RNA instability motifs, splice-activating motifs, polyadenylation motifs, adenine-rich sequence steps, endonuclease recognition sites and the like.

Preferably, the nucleic acid optimisation comprises optimising in each case the codon choice for the desired expression system.

In addition, it is possible to raise or reduce expression levels of a gene product, utilizing nucleic acid optimization methods in addition to the insertion of CpG dinucleo tides, for example by modifying the codon choice. Expression-optimised constructs according to the invention can be produced for example by choosing the codon distribution to be the same as in the expression system that is used.

Preferably, the eukaryotic expression system is a mammalian system, for example a human system. The codon optimisation can be matched to the codon choice of human genes. A codon choice that is most frequently or next to most frequently employed in mammalian cells (Ausubel et al., 1994) can be used in order to ensure a general stabilisation of the RNA and an optimal codon choice.

The nucleic acid sequence can be modified for optimal expression by using a suitable gene optimiser technology, such as the ones detailed in patent publications DE 102 60 805.9 and PCT/EP03/14850.

Any suitable expression vector known in the art can be utilized within the system and method as described herein.

Preferably, the vector is preferably suitable for expression in eukaryotic cells.

The modified target nucleic acid sequence to be expressed can be cloned into the vector so that it is in operative coupling with a suitable transcription control sequence and possibly further regulator elements.

A suitable promoter, which may either be constitutive or inducible, may be such a transcription control sequence. Constitutively active promoters can be selected from, but are not restricted to, CMV (Cytomegalovirus) promoter and Simian Virus 40 (SV40). Inducible promoters include, but however are not restricted to, tetracyclin-dependent promoters. Further suitable promoters known in the art can be selected depending on the application, e.g. promoters of particular cellular origin.

Any inducible promoter system that is known in the prior art can be utilzied. For example, a natural or artificial inducible promoter may be used, for example a promoter inducible by tetracyclin (Tet on/Tet off system). An inducible viral promoter may also be used.

The inducible promoter can be induced by a trans-active factor. A viral inducible promoter which can be induced by a viral trans-active factor may be derived from an arbitrary virus. Sequences of retroviruses, HCV (Hepatitis C virus), HBV (Hepatitis B virus), HSV (Herpes Simplex virus), EBV (Epstein-Barr virus), SV 40 (Simian virus 40), AAV (Adena- associated virus), Adenovirus, Papilloma viruses or Ebola virus are can be used for the derivation purpose.

The trans-active factors can be selected for example from the following viral factors, but are not restricted to these: NS5A (HCV), HB X (HBV), VP16/ICP4 (EBV), EBNAl/Rta (EBV), ART (HHV8), Large T-Antigen (SV40), Rep78/68 (AAV), E1A (Adenovirus), E2 (Papilloma virus) and VP30 (Ebola virus).

An inducible promoter that can be induced by a viral trans-active factor can be utilized. For example, a retroviral LTR promoter or a functional partial sequence thereof can be used. The trans- active factor can be a retroviral Tat or Tax protein. The LTR promoter may be selected from the LTRs of HIV-1, HIV-2, SIV, HTLV and other related retroviruses that have LTR promoters. In particular, lentiviral promoters can be utilized, such as those of HIV.

Preferably, the transcription control sequences, i.e. for example promoters and/or enhancers, etc., used within the scope of the present invention are not associated with CpG islands.

In addition to increasing the number of CpG dinucleotides in the target nucleic acid to be expressed, to reduce the number of CpG dinucleotides in the remaining sequences or parts thereof present on the nucleic acid or the vector as a whole. For example, the CpG dinucleotides in these remaining sequences or parts thereof may be partially completely eliminated.

Preferably, the elimination is again carried out while retaining the amino acid sequence in a gene producrt by utilising the degeneracy of the genetic code.

Preferably, only a partial elimination of the CpG dinucleotides in these sequences may take place, for example of at least 5%, preferably at least 10%, more preferably at least 15%, particularly preferably at least 25%, more particularly preferably 50%, and most particularly preferably 75% or more. In some embodiments, all CpGs are removed insofar as this is possible. Depending on the application (silencing or increasing the expression) the number of CpG dinucleotides may be varied independently of the chosen codon optimisation. In many cases, a complete elimination of CpGs from the reading frame is possible. The coded amino acid sequence can be upwardly limiting, i.e. as regards increasing the number of CpGs. Methods optimizing CpG dinucleotide content in nucleic acids are further described in detail in US Pat. Pub. 2009/0324546.

### Exemplary Module 4: DETECTING ERRORS IN NUCLEIC ACID CONSTRUCTS

The methods and systems as described herein allow for error checking in designed nucleic acid constructs.

The system and method as described relates to one or more growing databases comprising data and/or metadata for individual genetic objects.

Preferably, the database comprises dependency information for genetic objects.

System and method as described herein may compare designed nucleic acid constructs to database entries by sequence alignment.

The determination for the presence of an error with above threshold likelihood can be based on the sequence alignment to the database entry, as well as the dependency information.

The system and method as described herein relate to flagging portions or an entirety of a designed nucleic acid constructs, when algorithms comparing the constructs to database entries output above a threshold likelihood of errors.

Preferably, the systems and methods provide an output with an alteration of the designed nucleic acid construct.

Preferably, the alteration would fix a determined error in the designed nucleic acid construct.

Preferably, the methods and systems as described herein automatically accept the alteration and update the nucleic acid construct.

Preferably, a user can be given the option to accept the alteration.

Preferably, the methods and systems as described herein comprise a module for debugging of designed DNA sequences. Based on growing database of metadata for each genetic "object"- a dependency fields in the metadata and sequence alignment + dependency check for each object+ a flagging and possible "fix" suggestion.

### Exemplary Module 5: DESIGNATING METABOLIC LABELS TO NUCLEIC ACID CONSTRUCTS

Generally, metabolic pathways comprise networks of molecules connected by enzymes that catalyze the formation of one molecule from the next.

Metabolic engineering of cells to produce a specific molecule (drug, fuel etc) is a common use of synthetic biology.

Localizing information and visualizing some or all of the metabolic pathways in a cell and/or organism, or linking metabolic pathways to enzymes and/or to nucleic acids encoding the same, would be an extremely useful tool for people in the field of synthetic biology.

In addition one or more metabolic pathway descriptions are annotated to a designed nucleic acid construct.

Preferably, the annotation is aided by a metabolic database comprising (E.C.) numbers related to genetic objects.

The E.C. number is a numerical classification scheme for enzymes, based on the chemical reactions they catalyze. In this system of enzyme nomenclature, every EC number is associated with a recommended name for the reaction catalyzed by respective enzyme. Thus, E.C. numbers specify enzyme-catalyzed reactions. Two different enzymes (for instance from different organisms) catalyzing the same reaction can receive the same E.C. number

Preferably, the E.C. numbers are obtained from a genome file related to the genetic object.

A portion or the entirety of a nucleic acid construct can be aligned against the genetic objects in the metabolic database.

Genetic objects carrying above a designated threshold level of % similarity or identity to sequences with annotated metabolic pathways upon alignment can be annotated with the same or similar metabolic pathway description.

Generally, some or all genes and/or genetic objects in the nucleic acid construct, for example a target genome, can be sent to an alignment module, such as the NCBI Blast tool (http://blast.ncbi.nlm.nih.gov/Blast.cgi). A nucleic acid alignment algorithm, such as the Megablast algorithm, can be applied to the submitted sequences to find sequences from a database with a high alignment score, for example greater than 95% similarity or identity.

The annotated E.C. number associated with the picked sequence can be assigned to the submitted gene or genetic object.

Preferably, the assignment of the E.C. numbers is automated.

Any assigned E.C. numbers to the designed nucleic acid constructs can be stored in an associated file, e.g. a project file and/or database.

Metabolic pathways with the listed E.C. numbers in a nucleic acid construct project can be displayed in various ways. For example, an image/cartoon of the pathway can be displayed automatically or upon user query.

Preferably, the user query comprises hovering over a user interface object with a mouse or clicking a user interface object with a mouse.

In another example, catalytic activities can be classified into groups. Catalytic activity groups can be displayed using various visual icons associated with a gene/genetic object.

Preferably, the systems and methods leverage metabolic pathways by processing pathways related to an end or intermediate product of the pathway.

For example, a user can select a metabolic product and/or an enzyme in a pathway.

An algorithm can determine various metabolic pathway possibilities for the production of the selected metabolic product.

The algorithm can further provide options comprising one or more genes and/or genetic elements to increase or decrease the production, decay and/or accumulation of the selected metabolic product.

In another example, the functions of a selected enzyme can be determined across multiple metabolic pathways and replacement possibilities can be offered as options comprising one or more genes and/or gene products.

Preferably, the systems and methods comprise an algorithm for constructing an editable metabolic pathway description of each design "on the fly". Works by harvesting E.C number metadata from the genome file or from each genetic "object" combined with sequence alignment of all the genes/objects in the design with the gen bank database and automatically assigning E.C number to un-annotated genes.

### Exemplary Module 6: OPTIMIZING THE SYNTHESIS OF NUCLEIC ACID CONSTRUCTS. MINIMIZING COST

The system and method as described herein relate to the synthesis of nucleic acid constructs, such as those designed using the systems and methods as described.

Preferably, the systems and methods comprise a database for stock sequences and/or previously ordered sequences.

A nucleic acid construct can be checked against the database to determine whether portions or the entirety of the construct align to the entries of the database at above a threshold identity level.

For example, a one base-pair difference between a construct and database entry can lead to the manufacturing of the desired construct with ease by replacing the single base-pair with the desired base- pair. In another example, many portions of a desired construct sequence can be located in the database, thus enabling the synthesis of the construct by assembling the shorter sequences.

Preferably, the optimized synthesis of a construct can be based on cost, ease, and/or speed.

Preferably, the price of the synthesis can be adjusted based on a selected synthesis method.

The synthesis optimization can be adapted to different criteria by a user, for example, a more expensive synthesis method can be elected based on its desired speed.

Preferably, the systems and methods comprise an algorithm for compiling a design in the cheapest way to the user and/or the higher margin for us. Checking our database for already ordered parts and shorter DNA snippets (if already ordered- much higher margin/price for reordering parts) and designing the most cost effective order possible.

### Exemplary Module 7: GENERATING SYNTHESIS PROTOCOLS FOR NUCLEIC ACID CONSTRUCTS

The system and method as described herein relate to systems and methods generating nucleic acid synthesis protocols for nucleic acids, for example designed nucleic acid constructs generated using the systems and methods as described.

Preferably, an algorithm automatically generates an automated construction protocol for nucleic acids, for example a genome or a plasmid. For example, an algorithm can generate a construction protocol with various criteria, such as cost, speed, and/or ease.

Preferably, the systems and methods comprise an algorithm for creating an automated genome/plasmid construction protocol based on the cheapest/fastest/easiest construction method possible (depending on the user preferences and parts compiling results)

### Exemplary Module 8: FEATURES OF THE INTERACTIVE USER INTERFACE

The system and method as described herein, relate to the interactive user interface to visualize biological information relating to design operations.

Preferably, the user interface comprises plurality of resolution layers.

Resolution layers can display information in a relevant way according to a chosen "zoom" level.

Preferably, the resolution layers correspond to differential visual representation of biological information.

Preferably, the user interface can dynamically switch visual representation in relation to a "zoom" level.

Preferably, the biological information originates from a database that is operably linked to the systems of the invention.

Preferably, the various visual representations of biological information relate to an abstraction level. The abstraction levels can be chosen to optimally represent biological information in desired sufficiency, for example various abstraction levels of the invention can correspond to organism, genome, system, metabolic pathway, protein, genetic sites, amino acids, or nucleic acid level detail.

Preferably, the user interface comprises visual icons that can be selected by a user to switch to a desired abstraction layer. Accordingly, a corresponding "zoom" level can be displayed on the user interface.

Preferably, genetic elements, such as elements of the designed nucleic acid construct, can be "dragged" between zoom levels. For example, a genetic element can be selected by a computer mouse and be moved onto an icon representing the desired zoom level on the user interface.

Such an operation can switch the visualization of the genetic interface with the appropriate resolution, detail, and information. For example, upon switching to a metabolism view, the user interface can display a genetic object as an element of a metabolic pathway, if a related gene product participates in the metabolic pathway.

Preferably the dynamic zoom function of the systems and method as described herein, switches the display on a user interface between various views, such as gene view, metabolic simulation view, protein view, cellular view, organism view, organ view, complex organism view, for example yeast, plant, animal, human, gender specific view or any other suitable view known in the art.

Preferably, the user interface can display nucleic acid constructs in comparison to an average or most common species sequence.

Preferably, individual sequence variation in a nucleic acid sequence within a species can be displayed. A "normal" sequence can be designated, for example when a specific sequence variation is observed the majority of the time. In comparison to the "normal" sequence, individual sequences, mutations, for example cancer related mutations, can be displayed.

A sequence variation can be displayed as a phenotype; for example a resulting phenotype can be displayed using a "zoomed out" look at an organism level or at a live culture level, such as displaying a type of beer or bread affected by the live culture. Various views can display specific individuals in comparison to families, strains, genders, ethnicity, characteristic groups and any other suitable comparison object known in the art.

In one non-limiting example, a user can start from the top zoom level, where biological information is displayed using representative icons only. In some embodiments, the system comprises a G U.I with a zoom function for each abstraction layer: Organism, Genome, System, Sites, Amino Acids, and DNA. Ability to drag and drop genetic elements between zoom levels. Zoom levels description changes automatically to reflect the focused object (Organism, Gene, Metabolic Pathway, and Protein/DNA Structure) Zoom molecular/sequence code to gene view, metabolic simulation view, protein view, cellular view, organism view, organ view, complex organism view yeast, plant, animal, human, gender, generic species/compare individual sequence view, compare normal/modified view (mutation/cancer/other edits),
Zoom out to plants animals, foods with live culture (beer, bread), specific individuals, families, strains, genders, ethnicity, characteristic groups etc

Icons representing genes or genomes can be dragged and dripped into a design canvas.

For example, when E. coli genome is introduced into the design canvas, the software tool can read the genome and display the genome properties and data visualization tools, such as the metabolic pathway tool, that allow users to easily design features in light of the pathway for the production of different bio-chemicals.

Various modules may allow molecular modeling and/or simulations of protein structure. When we zoomed in to the genome, genes and/or genetic elements can be easily and accurately moved, annotated, mutated and/or deleted.

### Exemplary Module 9: TRANSACTIONS MODULE

Preferably, nucleic acid constructs, can be ordered purchased using the methods and systems as described.

Preferably, the nucleic acid construct is designed using the methods and systems as described.

Preferably, a marketplace of nucleic acid constructs populates constructs available for purchase. The marketplace can be populated by nucleic acid synthesis vendors and/or individual users.

A user can submit a constructs with sequence information, as well as any other relevant information, such as any gene products, organism preferences, metabolic pathways or any other suitable information known in the art. In some embodiments, user can review and/or rank the constructs in the marketplace.

User participation can be encouraged by incentives, such as offers of revenue share from ordered constructs.

Constructs that are repeatedly ordered can be price adjusted, for example in a way that protects profit margins while benefiting from decreased manufacturing costs.

The purpose of a designed construct can play a role determining a price for using the design tools and/or the synthesized product. For example, use of the design software to generate constructs that are likely to be useful in a cancer pathway may be more expensive or cheaper than a use related to agriculture.

Constructs in the marketplace can be linked by user behavior, for example, constructs that are ordered by a shared user increase the relatedness of the constructs.

Constructs above a threshold level of relatedness to a specific construct can be suggested to the user of the system based on the user's interest in the specific construct.

Preferably, the methods and systems as described allow for the creation of a marketplace for nucleic acids constructs. For example, users can easily design the DNA source code and order related products selecting from a variety of prices allowing for the election of a best price.

For another example, DNA synthesis vendors can be offered large and predictable order volume through the use of the marketplace.

Preferably, the methods and systems as described allow for the use of software tools at various user and/or pricing levels. For example, enhanced users, such as academic pro users or industrial users can be asked to pay a subscription fee for advanced features not available in lower cost versions of the software.

Preferably, different versions of the design tools are made available to multiple tiers of users. For example, a four-tier user model may comprise an "open" user, a registered user, an academic pro user, and industry user.

An "open user" tier may be set up not requiring any login information, but offering the option for saving and/or ordering a design.

Users in the registered user tier can order designs through the system. Real life information, such as an address and/or credit card number may be required for registered users. Registered users may be allowed to save more designs and post ordered designs in the marketplace. Academic pro users, for example users working in research laboratories, may be offered premium costumer support, for example with customer support agents with advanced degrees, such as Ph.D.s in relevant sciences. The academic pro users can be given access to a professional desktop application, optionally with extra features and/or unlimited storage place for their designs. An industry user can be offered private and secure data storage at a relevant price level.

Preferably, the systems and methods relate to the generation of a biological application store described herein. For example, it is generally expensive to synthesize DNA the first time, but once a particular construct is synthesized, DNA can be replicated and maintained for orders of magnitude cheaper.

Users can be incentivized to curate and post their ordered designs in our store. The users can be offered a revenue stream from the ordering of their designs. The more a posted design allows users to utilize the design cheaper, easier, and more likely to work, the higher the likelihood of it being ordered, thus posters of designs are encouraged to provide related information to allow for increased orders. In various embodiments, the curated user posted designs allow for increases in repeat orders and further, higher profit margins.

### Exemplary Module 10: COMPILATION MODULE

Preferably the systems comprise a compilation module adapted for checking a designed nucleic acid sequences against a list of predetermined rules.

Preferably, the compilation module is further configured to execute internally in the client, and/or in a server.

Preferably, the compilation module comprises a list of design rules.

Preferably, the design rules are based on client rules and/or system rules.

Preferably, the compilation module is further configured to enable a user to add his own defined rules to said list.

Preferably, the compilation module is further configured to send a user a list of possible problems found within his designed construct with visual warning about the location and the type of the possible problem.

Preferably, the compilation module is further configured to enable a user to review and linked directly to the location of said possible problem

Preferably, the compilation module is further adapted for to enabling a user to change said problem and/or ignore the warning.

Preferably, the list of rules is compiled by Genome Compiler

Preferably, the list of rules is based on client rules thereby, the user can add his own user defined rules to the list.

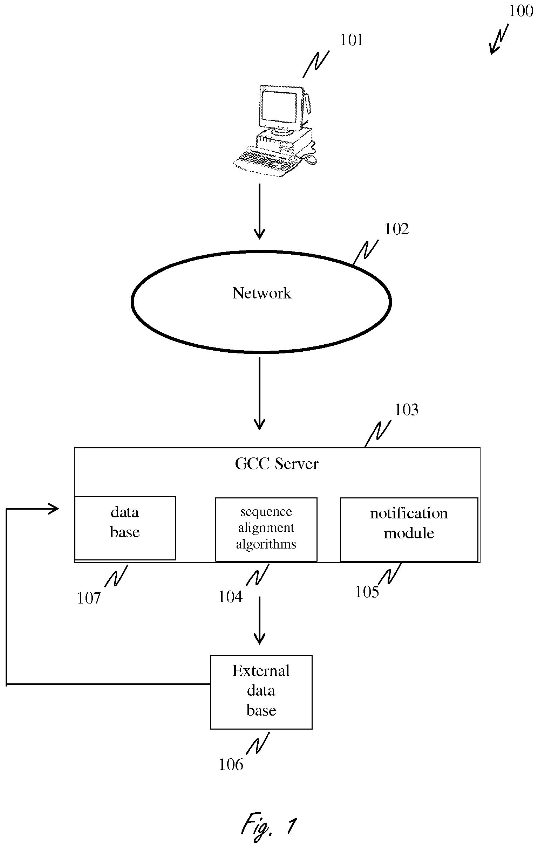

**Reference is now made to** **Fig. 1****,** which illustrate in a non-limiting manner the detecting module for detecting of designated sequences including harmful sequences, in the system as described above.

Preferably, each time a user save, import or edit a DNA record on his computer [101], the file and the use's logs are sent over a network [102] to Genome Compiler Servers - GCC server [103] and that sequence is aligned via sequences alignment module [104] by sequence alignment algorithms module (both possible proteins/reading frames and the DNA) to a database of harmful organisms and toxins [107].

The server [103] notify using notification module [105] to Genome Compiler system whenever a user from a specific LP address (if the user logging in - also who the user is) is working on a dangerous toxin.

A human operator then goes through the logs and constructs to see if it is malicious intent, or an acceptable research from a reputable institution. If a problem is found then Genome Compiler will notify the authorities.

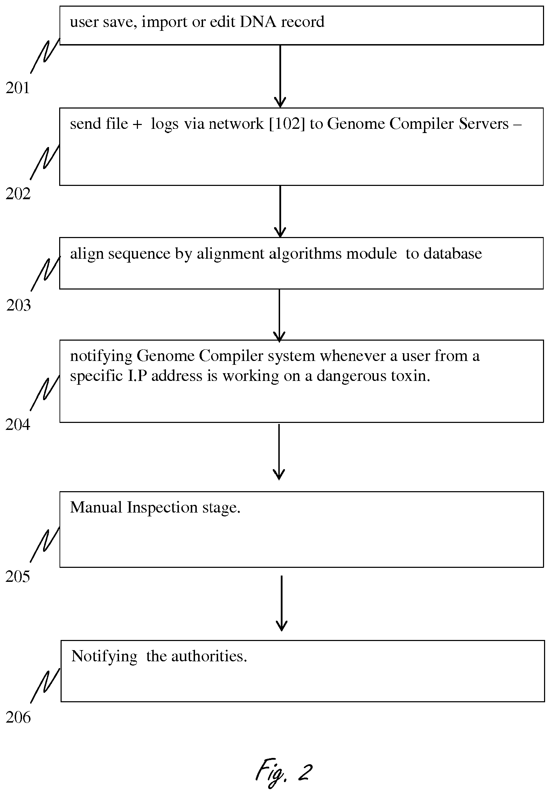

**Reference is now made to** **Fig. 2****,** which illustrate in a non-limiting manner the above detecting module operating in a method comprising steps of:
**step 201:** a user save, import or edit a DNA record on his computer [101]; **step 202** sending the file and the use's logs over a network to Genome Compiler Servers - GCC server; **step 203** aligning the sequence by sequence alignment algorithms module (both possible proteins/reading frames and the DNA) to a database of harmful organisms and toxins; **step 204** notifying Genome Compiler system whenever a user from a specific LP address (if the user logging in - also who the user is) is working on a dangerous toxin.

**Step 205** a human operator then go through the logs and constructs to see if it is a malicious intent, or an acceptable research from a reputable institution. **Step 206** If a problem is found then Genome Compiler will notify the authorities.

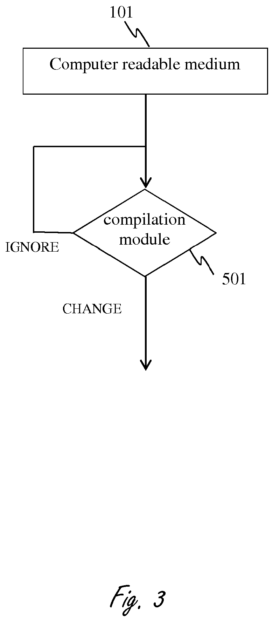

**Reference is now made to** **Fig. 3** which illustrate in a non-limiting manner the computer readable medium [101] comprising a compilation module [501] adapted for functionally checking a designed nucleic acid sequences against a list of predetermined rules.

Preferably, each time a user change and/or edit a project, the sequence file is checked (internally in the client [101] and/or in via network in the server [103]) against a list of design rules and send the user a list of possible problems with a visual warning about the location and the type of possible problem.

The user can then review and linked directly to the location of the possible problem where he can change or ignore the warning.

Preferably, the list of rules is compiled by Genome Compiler

Preferably, the list of rules is based on client rules thereby, the user can add his own user defined rules to the list.

## Claims

1. A computerized system for designing and synthesizing nucleic acid sequences for gene expression comprising;
a. a server (103) for hosting a database (107), wherein the database (107) comprises a plurality of entries, each entry comprising a nucleic acid sequence encoding for a harmful gene expression product, wherein the nucleic acid sequence encoding for the harmful gene expression product is from a bacterial genome or a viral genome;
b. a network connection (102)
c. a computer readable medium (101), wherein said computerized system is configured for operating in a method of:
i.) visualizing biological information in an interactive user interface relating to design instructions;
ii.) designing nucleic acid sequences;
iii.) automatically flagging when the nucleic acid sequence encoding for the harmful gene expression product is included in the nucleic acid sequence for at least one gene;
iv.) automatically modifying the interactive user interface to notify when the nucleic acid sequence encoding for the harmful gene expression product is detected in the nucleic acid sequence for at least one gene;
v.) providing an alternative alteration of the designed nucleic acid sequences, wherein the alternative alteration does not encode for the nucleic acid sequence encoding for the harmful gene expression product; and
vi.) synthesizing a nucleic acid encoding for the alternative alteration of the designed nucleic acid sequences.

2. The system of claim 1, wherein said computerized system is further configured for optimizing said designed nucleic acid construct according to various requirements, wherein said various requirements are selected from a group consisting of: optimizing codon usage, optimizing GC content, optimizing CpG content, avoiding problems associated with DNA motifs, avoiding problems associated with sequence repeats, avoiding problems associated with inverse complementary sequence repeats and any combination thereof.

3. The system of claim 1, wherein said computerized system is configured to check the alignment level of designed nucleic acid constructs to said database (107) entries through sequence alignment..

4. The system of claim 3, wherein said computerized system is configured to provide output with the alternative alteration of said designed nucleic acid sequences.

5. The system of claim 1, wherein said computerized system is further configured for optimizing said synthesis of the nucleic acid sequences.

6. The system of claim 5, wherein said optimizing is based on cost, ease, and/or speed.

7. The system of claim 1, wherein said interactive user interface is further configured to display various modules and, wherein said various modules are selected from a group consisting of nucleic acid design, error checking, codon optimization, metabolic product design, visual representations of biological information with dynamic zoom, nucleic acid synthesis, transactions regarding ordering and/or purchasing synthesized nucleic acids, user review, and ranking of nucleic acid constructs.

8. The system of claim 1, wherein said computerized system is further configured for design of expressions constructs based on a list of design rules.

## Patentansprüche

1. Computergestütztes System zum Entwerfen und Synthetisieren von Nukleinsäuresequenzen für die Genexpression, umfassend:
a. einen Server (103) zum Hosten einer Datenbank (107), wobei die Datenbank (107) eine Vielzahl von Einträgen umfasst, wobei jeder Eintrag eine Nukleinsäuresequenz umfasst, die für ein schädliches Genexpressionsprodukt kodiert, wobei die Nukleinsäuresequenz, die für das schädliche Genexpressionsprodukt kodiert, von einem bakteriellen Genom oder einem viralen Genom stammt;
b. eine Netzwerkverbindung (102)
c. ein computerlesbares Medium (101), wobei das computergestützte System ausgelegt ist für den Betrieb in einem Verfahren für
i.) die Visualisierung biologischer Informationen in einer interaktiven Benutzerschnittstelle in Bezug auf Entwurfsanweisungen;
ii.) das Entwerfen von Nukleinsäuresequenzen;
iii.) das automatische Signalisieren, wenn die Nukleinsäuresequenz, die für das schädliche Genexpressionsprodukt kodiert, in der Nukleinsäuresequenz für mindestens ein Gen enthalten ist;
iv.) das automatische Modifizieren der interaktiven Benutzerschnittstelle zur Benachrichtigung, wenn die Nukleinsäuresequenz, die für das schädliche Genexpressionsprodukt kodiert, in der Nukleinsäuresequenz für mindestens ein Gen nachgewiesen wird;
v.) das Bereitstellen einer alternativen Veränderung der entworfenen Nukleinsäuresequenzen, wobei die alternative Veränderung nicht für die Nukleinsäuresequenz kodiert, die für das schädliche Genexpressionsprodukt kodiert; und
vi.) die Synthese einer Nukleinsäure, die für die alternative Veränderung der entworfenen Nukleinsäuresequenzen kodiert.

2. System nach Anspruch 1, bei dem das computergestützte System des Weiteren derart konfiguriert ist, dass es das entworfene Nukleinsäurekonstrukt verschiedenen Anforderungen gemäß optimiert, wobei die verschiedenen Anforderungen ausgewählt sind aus einer Gruppe bestehend aus: Optimierung der Codon-Nutzung, Optimierung des GC-Gehalts, Optimierung des CpG-Gehalts, Vermeidung von Problemen im Zusammenhang mit DNA-Motiven, Vermeidung von Problemen im Zusammenhang mit Sequenzwiederholungen, Vermeidung von Problemen im Zusammenhang mit inversen komplementären Sequenzwiederholungen, und jedweder Kombination dieser.

3. System nach Anspruch 1, bei dem das computergestützte System derart konfiguriert ist, dass es den Alignment-Level von entworfenen Nukleinsäurekonstrukten zu den Einträgen der Datenbank (107) durch Sequenzalignment überprüft.

4. System nach Anspruch 3, bei dem das computergestützte System derart konfiguriert ist, dass es als Ausgabe die alternative Veränderung der entworfenen Nukleinsäuresequenzen liefert.

5. System nach Anspruch 1, bei dem das computergestützte System des Weiteren derart konfiguriert ist, dass es die Synthese der Nukleinsäuresequenzen optimiert.

6. System nach Anspruch 5, bei dem diese Optimierung auf der Grundlage von Kosten, Einfachheit und/oder Geschwindigkeit erfolgt.

7. System nach Anspruch 1, bei dem die interaktive Benutzerschnittstelle des Weiteren derart konfiguriert ist, dass sie verschiedene Module anzeigt, und bei dem die verschiedenen Module aus einer Gruppe ausgewählt sind, die aus dem Entwerfen von Nukleinsäure, Fehlerprüfung, Codon-Optimierung, dem Entwerfen von Stoffwechselprodukten, visuellen Darstellungen biologischer Informationen mit dynamischem Zoom, Nukleinsäure-Synthese, Transaktionen bezüglich des Bestellens und/oder des Kaufes synthetisierter Nukleinsäuren, Benutzerbewertung und Ranking von Nukleinsäurekonstrukten besteht.

8. System nach Anspruch 1, bei dem das computergestützte System des Weiteren für den Entwurf von Expressionskonstrukten auf der Grundlage einer Liste von Entwurfsregeln konfiguriert ist.

## Revendications

1. Système informatisé pour la conception et la synthèse de séquences d'acide nucléique pour l'expression génique, comprenant :
a. un serveur (103) destiné à héberger une base de données (107), la base de données (107) comprenant une pluralité d'entrées, chaque entrée comprenant une séquence d'acide nucléique codant pour un produit d'expression génique pathogène, la séquence d'acide nucléique codant pour le produit d'expression génique pathogène provenant d'un génome bactérien ou d'un génome viral ;
b. une connexion à un réseau (102)
c. un support lisible par ordinateur (101), ledit système informatisé étant conçu pour une exploitation dans une méthode de :
i.) visualisation d'informations biologiques dans une interface utilisateur interactive concernant des instructions de conception ;
ii.) conception de séquences d'acide nucléique ;
iii.) signalisation automatique quand la séquence d'acide nucléique codant pour le produit d'expression génique pathogène est incluse dans la séquence d'acide nucléique pour au moins un gène ;
iv.) modification automatique de l'interface utilisateur interactive pour notifier quand la séquence d'acide nucléique codant pour le produit d'expression génique pathogène est détectée dans la séquence d'acide nucléique pour au moins un gène ;
v.) fourniture d'une altération alternative des séquences d'acide nucléique conçues, l'altération alternative ne codant pas pour la séquence d'acide nucléique codant pour le produit d'expression génique pathogène ; et
vi.) synthèse d'un acide nucléique codant pour l'altération alternative des séquences d'acide nucléique conçues.

2. Système selon la revendication 1, ledit système informatisé étant en outre conçu pour optimiser ladite construction d'acide nucléique conçue selon diverses exigences, lesdites diverses exigences étant choisies dans un groupe consistant en : l'optimisation de l'usage des codons, l'optimisation de la teneur en GC, l'optimisation de la teneur en CpG, l'évitement des problèmes liés aux motifs d'ADN, l'évitement des problèmes liés aux répétitions de séquences, l'évitement des problème liés aux répétitions de séquences complémentaires inverses, et toute combinaison de ceux-ci.

3. Système selon la revendication 1, ledit système informatisé étant conçu pour vérifier le niveau d'alignement des constructions d'acide nucléique conçues sur lesdites entrées de base de données (107) grâce à l'alignement de séquence.

4. Système selon la revendication 3, ledit système informatisé étant conçu pour fournir une sortie avec l'altération alternative desdites séquences d'acide nucléique conçues.

5. Système selon la revendication 1, ledit système informatisé étant en outre conçu pour optimiser ladite synthèse des séquences d'acide nucléique.

6. Système selon la revendication 5, dans lequel ladite optimisation est basée sur le coût, la facilité et/ou la vitesse.

7. Système selon la revendication 1, dans lequel ladite interface utilisateur interactive est en outre conçue pour afficher divers modules et, dans lequel lesdits divers modules sont choisis dans un groupe consistant en la conception d'acides nucléiques, la vérification d'erreurs, l'optimisation des codons, la conception de produits métaboliques, les représentations visuelles de l'information biologique avec un zoom dynamique, la synthèse d'acides nucléiques, les transactions concernant la commande et/ou l'achat d'acides nucléiques synthétisés, la revue d'utilisateurs, et le classement des constructions d'acides nucléiques.

8. Système selon la revendication 1, ledit système informatisé étant en outre conçu pour la conception de constructions d'expressions sur la base d'une liste de règles de conception.
